# EUROPÄISCHE PATENTSCHRIFT

(11) **EP 0 915 084 B1**
(45) Veröffentlichungstag und Bekanntmachung des Hinweises auf die Patenterteilung: **23.04.2003**
(21) Anmeldenummer: 98119420.2
(22) Anmeldetag: 14.10.1998
(51) Int. Cl.: C07C 277/08, C07C 275/70, C07C 279/14

(54) **Verfahren zur Herstellung von Kreatin**
Process for the preparation of creatine
Procédé pour la préparation de la créatine

(30) Priorität: 04.11.1997 DE 19748695
(43) Veröffentlichungstag der Anmeldung: 12.05.1999
(73) Patentinhaber: BASF AKTIENGESELLSCHAFT, 67056 Ludwigshafen (DE)
(72) Erfinder: Greindl, Thomas Dr., 67098 Bad Dürkheim (DE); Scherr, Günter Dr., 67065 Ludwigshafen (DE); Schneider, Rolf Dr., 68163 Mannheim (DE); Mundinger, Klaus Dr., 67117 Limburgerhof (DE)

(56) Entgegenhaltungen:
- US-A- 2 727 922
- DATABASE CHEMABS [Online] CHEMICAL ABSTRACTS SERVICE, COLUMBUS, OHIO, US STN, accession no. 94:174389, XP002117305 & JP 55 151547 A (PERMACHEM ASIA LTD)
- P. LUGOSI ET AL: PERIOD. POLYTECH., CHEM. ENG., Bd. 19, Nr. 4, 1975, Seiten 307-316, XP002117424

## Beschreibung

Die Erfindung betrifft ein Verfahren zur Herstellung von Kreatin durch Umsetzung von Haloformamidiniumsalzen mit Sarkosin.

Substituierte Guanidiniumverbindungen sind in der Natur weit verbreitet. Wichtige Vertreter dieser Substanzklasse sind beispielsweise Aminosäuren, wie Arginin und Kreatin. Darüberhinaus sind substituierte Guanidinderivate als sterisch gehinderte Basen, als Biozide sowie als Komplexliganden bekannt. Die Mehrzahl der Verbindungen dieses Typs sind aufgrund der hohen Herstellkosten in ihrer technischen Anwendbarkeit jedoch stark eingeschränkt.

Ein Beispiel für ein biologisch aktives Guanidinderivat ist Kreatin, das als "Energieträger der Zelle" zur Nahrungsergänzung im Food und Pharmabereich eingesetzt wird.

Die Herstellung von Kreatin wird z.B. in EP-A-O 754 679 und der dort zitierten weiterführenden Literatur beschrieben, wobei maximale Ausbeuten von lediglich 70% erzielt werden. Ein Nachteil dieser Synthese für Guanidiniumverbindungen ist, daß in allen Fällen wässrige Lösungen von reinem Cyanamid eingesetzt werden. Diese Lösung ist jedoch sehr teuer und aufgrund der Instabilität des Cyanamids im allgemeinen nicht breit verfügbar.

Alternative Synthesen von Guanidiniumsalzen verlaufen unter Verwendung von O-Alkylisoharnstoffen bzw. O-Alkylisothioharnstoffen. So wird in JP 55 004 349 eine Lösung von Na-Sarkosinat mit O-Methylisoharnstoff-Methylsulfat in Ausbeuten von 80% zum Kreatin umgesetzt. All diese Synthesen machen den Einsatz von Alkylierungsmitteln wie z.B. Dimethylsulfat erforderlich und sind aufgrund auftretender Mehrfachalkylierungen häufig nicht ausreichend selektiv.

Es bestand daher die Aufgabe, ein Verfahren zur Herstellung von Kreatin bereitzustellen, das die oben genannten Nachteile nicht aufweist.

Diese Aufgabe wurde gelöst durch ein Verfahren zur Herstellung von Kreatin, durch
a) Umsetzung von Kalkstickstoff mit Salzsäure, Bromwasserstoffsäure oder Jodwasserstoffsäure oder Gemischen von Mineralsäuren zum Haloformamidiniumsalz der Formel II, 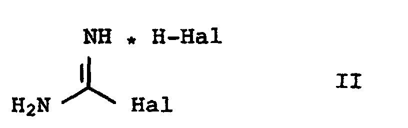 in der Hal = Cl, F, Br und J bedeuten kann, und
b) Umsetzung des gebildeten Haloformamidiniumsalzes der Formel II mit Sarkosin,
dadurch gekennzeichnet, daß das Haloformamidiniumsalz ohne Isolierung direkt mit Sarkosin umgesetzt wird.

Das erfindungsgemäße Verfahren erfüllt insbesondere wirtschaftliche Randbedingungen, wie niedrige Einsatzstoffkosten, technisch einfache Durchführbarkeit, verbesserte Ausbeuten und hinreichende Reinheit des Produktes.

Sarkosin kann sowohl als freie Säure als auch insbesondere als Na- oder K-Salz in Form einer 5 bis 60 Gew.-%igen, bevorzugt 35 bis 45 Gew.-%igen wäßrigen Lösung verwendet werden.

Die für die Synthese von Kreatin verwendeten Haloformamidiniumsalze sind in reiner Form stabile Verbindungen. Dies gilt insbesondere für das bevorzugt verwendete Chlorformamidin Hydrochlorid, das sowohl gemäß J. Am. Chem. Soc. 1992, 114, 9386-9390 aus reinem Cyanamid durch Umsetzung mit Chlorwasserstoff als auch durch Umsetzung des sehr billigen und wohlfeilen Calciumcyanamids (Kalkstickstoff) mit Salzsäure, gemäß US 2,727,922 beschrieben, gewonnen werden kann.

Gemäß US 2,727,922 kann zur Herstellung des Chlorformamidiniumchlorids anstelle des teuren Cyanamids sehr billiger und breit verfügbarer Kalkstickstoff eingesetzt werden. Unter Kalkstickstoff versteht man Produkte, die z.B. bei der Umsetzung von CaC₂ mit N₂ bei sehr hohen Temperaturen von 800 bis 1100°C entstehen. Sie enthalten in der Regel 5 bis 98 Gew.-%, bevorzugt 20 bis 95 Gew.-%, besonders bevorzugt 30 bis 90 Gew.-% Calciumcyanamid. Der technisch erhältliche graue bis schwarze Kalkstickstoff enthält neben Calciumcyanamid je nach Reinheitsgrad noch Verunreinigungen wie z.B. Kohlenstoff, Ca-Carbid, CaO sowie Spuren von Metallen, üblicherweise in Anteilen < 1%. Es kann selbstverständlich auch reines Ca-Cyanamid umgesetzt werden, besonders vorteilhaft, weil wesentlich wirtschaftlicher, ist aber der Einsatz von technischem Kalkstickstoff. Dieser wird vorzugsweise in einer Korngrößenverteilung von 1 bis 100 µm, vorzugsweise 20 bis 80 µm eingesetzt. Es kann aber auch gekörntes, gestrangtes oder anderweitig verdichtetes Material verwendet werden.

Ein besonderer vorteil des Verfahrens besteht darin, daß das in wäßriger Synthese aus Salzsäure und Kalkstickstoff entstandene Chlorformamidiniumchlorid nach Abtrennung des gebildeten Kohlenstoffs sowie der unlöslichen anorganischen Salze, ohne weitere Aufreinigung direkt weiter zum Kreatin umgesetzt werden kann.

Verwendet man an dieser Stelle statt Salzsäure Bromwasserstoffsäure oder Jodwasserstoffsäure, so erhält man die entsprechenden Brom- bzw. Jodformamidiniumsalze.

Die Synthese erfolgt in der Art und Weise, daß man Kalkstickstoff zu 1 bis 6, bevorzugt 2 bis 4 Äquivalenten Säure zudosiert. Insbesondere können auch Gemische von Mineralsäuren, wie z.B. Salzsäure/Schwefelsäure oder Salzsäure/Phosphorsäure in einem Verhältnis von 20/1 bis 5/1, insbesondere 15/1 bis 8/1 verwendet werden. Diese Gemische haben den Vorteil, daß in der Reaktionsmischung vorliegende Schwermetalle gleichzeitig ausgefällt werden.

Darüberhinaus kann es vorteilhaft sein, Komplexbildner wie Aminopolycarboxylate, beispielsweise EDTA oder Aminopolyphosphonate sowie zur Entfernung geruchsbildender Nebenprodukte Oxidationsmittel, beispielsweise H₂O₂ einzusetzen. Auf diese Weise wird die Reinheit des Produktes verbessert, ohne daß es zu Ausbeuteverlusten kommt.

Die Reinheit des gebildeten Haloformamidiniumsalzes läßt sich außerdem erhöhen, indem man die Durchführung der Reaktion unter vermindertem Druck, bevorzugt bei 100 bis 900 mbar, durchführt, um so leichtflüchtige Nebenprodukte, insbesondere leichtflüchtige Schwefelverbindungen abzutrennen.

Die Zugabe von Kalkstickstoff erfolgt üblicherweise in gleichmäßigen Portionen über einen Zeitraum von 0,5 bis 10 Std., insbesondere von 2 bis 5 Std., so daß die gewünschte Reaktionstemperatur durch Kühlung gehalten werden kann. Übliche Reaktionstemperaturen liegen im Bereich zwischen -10 und 100°C, insbesondere zwischen 0 und 40°C.

Nach Beendigung der Reaktion kann das gebildete Haloformamidiniumsalz nach Abtrennung anfallender anorganischer Nebenprodukte, wie z.B. CaSO₄, CaCl₂, CaPO₄ und Kohlenstoff direkt ohne weitere Aufreinigungsschritte mit Sarkosin zu Kreatin umgesetzt werden.

Die weitere Umsetzung des Haloformamidiniumsalzes mit Sarkosin kann in Wasser oder einem mit Wasser mischbaren Lösungsmittel oder einem Gemisch davon durchgeführt werden. Die Reaktion erfolgt in der Regel bei einem pH-Wert im Bereich des pK-Wertes des Amins, d.h. in einem Bereich zwischen pH 6 und 14, bevorzugt zwischen 8 und 12.

Zur Einhaltung des pH-Wertes können u.a. Basen wie NaOH, KOH, LiOH, Ca(OH)₂, Ba(OH)₂, Mg(OH)₂ oder Na₂CO₃ verwendet werden.

Die Reaktionstemperaturen liegen im Bereich zwischen 0 und 100°C, bevorzugt zwischen 10 und 80°C, besonders bevorzugt zwischen 20 und 70°C.

Die Zugabe der Reaktionspartner kann in beliebiger Reihenfolge erfolgen. Eine bevorzugte Ausführungsform ist die, bei der das Haloformamidiniumsalz, insbesondere Chlorformamidiniumchlorid, zu einer Lösung des Sarkosins zugetropft wird.

Die Zugabe kann sich über einen Zeitraum von 0,5 bis 10 Std., bevorzugt 0,5 bis 6 Std., besonders bevorzugt 1 bis 3 Std. erstrecken. Nach der Dosierung wird in der Regel 0,5 bis 10 Std., bevorzugt 1 bis 3 Std. nachgerührt.

Eventuell ausgefallene anorganische Nebenprodukte werden bei Temperaturen von 20 bis 100°C, insbesondere von 50 bis 90°C abgetrennt.

Das Molverhältnis von Haloformamidiniumsalz zu Sarkosin liegt im Bereich von 0,9 bis 5,0, vorzugswiese 1,0 bis 2,0.

Besonders hohe Umsätze können erzielt werden, wenn man beispielsweise das billige, technische Chloformamidiniumchlorid im Überschuß zum verwendeten Amin einsetzt, was im Falle von reinem Cyanamid oder reinem Chlorformamidiniumchlorid häufig unwirtschaftlich ist.

Ein besonderer Vorteil des Verfahrens ist es, daß aufgrund entfallender Reinigungsschritte die Ausbeuten an Kreatin bei Einsatz von rohem Haloformamidiniumsalz höher liegt als bei der Herstellung und weiteren Umsetzung von reinem Haloformamidiniumsalzen. Außerdem gestaltet sich das erfindungsgemäße Verfahren aufgrund der geringeren Anzahl an Verfahrensschritten technisch einfacher.

Die mit dem erfindungsgemäßen Verfahren erzielte Reinheit des isolierten Kreatin ist mit der aus reinen Ausgangsstoffen hergestelltem Material vergleichbar. Dies ist insbesondere auf die hohe Reinheit des technischen Haloformamidiniumsalzes zurückzuführen.

Das gebildete Kreatin Lässt sich in der Regel in an sich bekannter Weise isolieren. Im Falle von nach der Umsetzung bereits gebildeten kristallinen Endprodukten kann die Isolierung durch einfaches Filtrieren, Waschen und ggf. Umkristallisieren erfolgen.

In den folgenden Beispielen wird das Verfahren zur Herstellung von Kreatin näher erläutert.

### Beispiel 1: Herstellung von Kreatin

### Stufe 1: Herstellung von Chlorformamidiniumchlorid aus Kalk-Stickstoff

50 g Kalkstickstoff mit einem CaCN₂-Gehalt von 43 Gew.-% wurden innerhalb von 2 Std. unter Eiskühlung zu einem Gemisch aus 300 g 20 Gew.-%iger wässriger Salzsäure und 1 g 85 Gew.-%iger Phosphorsäure gegeben. Die Reaktionstemperatur wurde zwischen 5 und 10°C gehalten. Während der Zugabe von Kalkstickstoff wurde das Reaktionsgemisch gleichzeitig mit 50 l/Std. Luft begast. Im Anschluß an die Zugabe von Kalkstickstoff wurde die Suspension 5 Std. bei 5 bis 10°C nachgerührt und filtriert. Der Filterrückstand wurde mit insgesamt 150 ml Wasser gewaschen. Das vereinigte Waschwasser mit Mutterlauge zeigte einen Gehalt von 26,8 g Chlorformamidiniumchlorid (nach HPLC), entsprechend 89% Ausbeute.

### Stufe 2:

Die in Stufe 1 erhaltene wäßrige Lösung wurde innerhalb von 3 Std. bei 20 bis 30°C zu einer Mischung aus 55,4 g einer 40 Gew.-%igen wäßrigen Na-Sarkosinatlösung und 16 g 50 %iger Schwefelsäure getropft. Der pH-Wert der Reaktionslösung wurde mittels 50%iger NaOH bei 10 gehalten. Nach erfolgter Zugabe wurde 5 Std. bei Raumtemperatur nachgerührt, anschließend bei 60°C filtriert und das Filtrat bei max. 60°C im Vakuum auf ca. 50 bis 60% der Gesamtmasse eingeengt. Der nach dem Abkühlen ausgefallene Niederschlag wurde abfiltriert, mit 3 x 20 ml kaltem Wasser gewaschen und getrocknet. Man erhielt 22,6 g Kreatinmonohydrat mit einer Reinheit > 99%.

## Patentansprüche

1. Verfahren zur Herstellung von Kreatin, durch
a) Umsetzung von Kalkstickstoff mit Salzsäure, Bromwasserstoffsäure oder Jodwasserstoffsäure oder Gemischen von Mineralsäuren zum Haloformamidiniumsalz der Formel II, 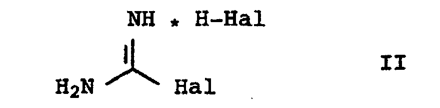 in der Hal = Cl, F, Br und J bedeuten kann, und
b) Umsetzung des gebildeten Haloformamidiniumsalzes der Formel II mit Sarkosin,
**dadurch gekennzeichnet, daß** das Haloformamidiniumsalz ohne Isolierung direkt mit Sarkosin umgesetzt wird.

2. Verfahren nach Anspruch 1, **dadurch gekennzeichnet, daß** man im Verfahrensschritt a) Kalkstickstoff mit Gemischen von Salzsäure und Schwefelsäure oder Salzsäure und Phosphorsäure in einem Verhältnis von Salzsäure zu Schwefelsäure oder Salzsäure zu Phosphorsäure von 20/1 bis 5/1 umsetzt.

3. Verfahren nach Anspruch 1, **dadurch gekennzeichnet, daß** man Sarkosin mit Chlorformamidiniumchlorid umsetzt.

4. Verfahren nach Anspruch 1, **dadurch gekennzeichnet, daß** man Sarkosin als Na- oder K-Salz in Form einer 5 bis 60 Gew.-%igen, wäßrigen Lösung verwendet.

## Claims

1. A process for preparing creatine by
a) reacting nitrolime with hydrochloric acid, hydrobromic acid or hydroiodic acid or mixtures of mineral acids to give the haloformamidinium salt of the formula II 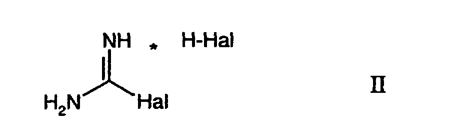 in which Hal can be Cl, F, Br and I, and
b) reacting the haloformamidinium salt of the formula II which has been formed with sarcosine,
wherein the haloformamidinium salt is reacted directly, without isolation, with sarcosine.

2. A process as claimed in claim 1, wherein nitrolime is reacted in step a) with mixtures of hydrochloric acid and sulfuric acid or hydrochloric acid and phosphoric acid in a ratio of hydrochloric acid to sulfuric acid or hydrochloric acid to phosphoric acid of from 20/1 to 5/1.

3. A process as claimed in claim 1, wherein sarcosine is reacted with chloroformamidinium chloride.

4. A process as claimed in claim 1, wherein sarcosine is used as Na or K salt in the form of a 5 to 60% by weight aqueous solution.

## Revendications

1. Procédé pour la préparation de créatine, par
a) réaction de chaux azotée ou cyanamide calcique avec de l'acide chlorhydrique, de l'acide bromhydrique ou de l'acide iodhydrique ou des mélanges d'acides minéraux pour former le sel d'halogénoformamidinium de formule II, 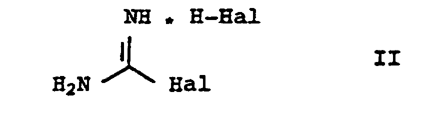 où Hal peut désigner Cl, F, Br et J, et
b) réaction du sel d'halogénoformamidinium de formule II obtenu avec de la sarcosine,
**caractérisé par le fait qu'**on fait réagir le sel d'halogénoformamidinium sans isolement, directement avec la sarcosine.

2. Procédé selon la revendication 1, **caractérisé par le fait que**, dans l'étape opératoire a), on fait réagir la chaux azotée avec des mélanges d'acide chlorhydrique et d'acide sulfurique ou d'acide chlorhydrique et d'acide phosphorique dans un rapport de l'acide chlorhydrique à l'acide sulfurique ou de l'acide chlorhydrique à l'acide phosphorique de 20/1 à 5/1.

3. Procédé selon la revendication 1, **caractérisé par le fait qu'**on fait réagir la sarcosine avec du chlorure de chloroformamidinium.

4. Procédé selon la revendication 1, **caractérisé par le fait qu'**on fait réagir la sarcosine à l'état de de sel de Na ou de K sous forme d'une solution aqueuse à 5 à 60% en poids.
